# EUROPEAN PATENT SPECIFICATION

(11) **EP 3 977 916 B1**
(45) Date of publication and mention of the grant of the patent: **14.10.2026**
(21) Application number: 20815081.3
(22) Date of filing: 29.05.2020
(51) Int. Cl.: G06V 10/82, G06V 10/40, G06T 7/00, G16H 15/00, G16H 30/40, G16H 50/20

(54) **MEDICAL DOCUMENT CREATION APPARATUS, METHOD AND PROGRAM, LEARNING DEVICE, METHOD AND PROGRAM, AND TRAINED MODEL**
VORRICHTUNG, VERFAHREN UND PROGRAMM ZUR ERSTELLUNG MEDIZINISCHER DOKUMENTE, LERNVORRICHTUNG, VERFAHREN UND PROGRAMM SOWIE TRAINIERTES MODELL
APPAREIL, PROCÉDÉ ET PROGRAMME DE CRÉATION DE DOCUMENTS MÉDICAUX, DISPOSITIF D'APPRENTISSAGE, PROCÉDÉ ET PROGRAMME, ET MODÈLE ENTRAÎNÉ

(30) Priority: 31.05.2019 JP 2019102059
(43) Date of publication of application: 06.04.2022
(73) Proprietor: FUJIFILM Corporation, Tokyo 106-8620 (JP)
(72) Inventor: MOMOKI, Yohei, Tokyo 106-8620 (JP)
(74) Representative: Klunker IP Patentanwälte PartG mbB
(86) International application number: PCT/JP2020/021431
(87) International publication number: WO 2020/241857

(56) References cited:
- EP-A1- 2 478 834
- JP-A- 2016 133 821
- JP-A- 2018 206 082
- JP-A- 2019 008 349
- US-A1- 2018 342 055

## Description

### BACKGROUND

### Technical Field

The present disclosure relates to a medical document creation apparatus, method and program, a learning device, method and program, and a trained model that create medical documents such as an interpretation report.

### Related Art

In recent years, advances in medical devices, such as computed tomography (CT) apparatuses and magnetic resonance imaging (MRI) apparatuses, have enabled image diagnosis using high-resolution medical images with higher quality. In particular, since a region of a lesion can be accurately specified by image diagnosis using CT images, MRI images, and the like, appropriate treatment is being performed based on the specified result.

Further, there are some cases in which a medical image is analyzed by computer-aided diagnosis (CAD) using a discriminator that has been trained by deep learning or the like, regions, positions, volumes, and the like of lesions included in the medical image are extracted to acquire these as the analysis result. In this way, the analysis result generated by the analysis process is saved in a database in association with examination information, such as a patient name, gender, age, and a modality which has acquired a medical image, and provided for diagnosis. A radiologist interprets the medical image by referring to the distributed medical image and analysis result and creates an interpretation report, in his or her own interpretation terminal.

Meanwhile, with the improvement of the performance of the CT apparatus and the MRI apparatus described above, the number of medical images to be interpreted is also increasing. However, since the number of radiologists has not kept up with the number of medical images, it is desired to reduce the burden of the image interpretation work of the radiologists. For this reason, various methods have been proposed to support the creation of medical documents such as interpretation reports. For example, JPWO2005-122002 proposes a method of specifying a template based on the input of findings and creating an interpretation report from the findings using the template in order to unify the terms and abbreviations used in the interpretation report. Further, JP2008-257569A proposes a method of performing language processing on a sentence based on information obtained by machine learning, extracting a plurality of elements belonging to each item including findings from the sentence, acquiring element-related information in association with the plurality of elements, displaying a list of information in which each element is associated with each other, and designating the plurality of elements as report components constituting report information according to a predetermined report model for each item included in the list display to generate a new report.

On the other hand, medical documents such as interpretation reports have different expression formats according to the features such as abnormal shadows included in the medical image. Therefore, it is preferable to create a medical document in an expression format according to the features included in the medical image. However, since the method described in JPWO2005-122002 is a method using a template, only a document having a uniform expression can be created even though the features included in the image are different. Further, it may not be possible to apply all the features included in the image to the template. Further, with the method described in JP2008-257569A, only an interpretation report of expressions according to a report model can be created. Therefore, the method described in JP2008-257569A may not be able to create a medical document suitable for the features included in the image.

EP2478834A1 discloses a radiograph interpretation report creation device, method, and program. A medical image is obtained and displayed. An electronic image interpretation report of the medical image is generated and displayed. The position of a lesion region in the medical image is input. A link character linked by a hyperlink to the medical image including the input position of the lesion region is inserted into the image interpretation report in such a manner that the link character is distinguishably displayed. The distinguishably displayed link character in the image interpretation report is selected. An index representing the position of the lesion region is displayed in the medical image based on the selection of the link character. US2018342055A1 discloses a medical scan assisted review system. JP2016133821A discloses an information processing device, information processing method, and program.

### SUMMARY OF THE INVENTION

The present disclosure has been made in view of the above circumstances, and an object thereof is to enable the creation of a medical document in an expression format according to the features included in a medical image.

In one aspect, there is provided a medical document creation apparatus according to claim 1 of the appended claims.

The "feature information including the feature vector of the target medical image" may be the feature vector of the target medical image itself, or may include accessory information of the target medical image in addition to the feature vector. As the accessory information, it is possible to use information that can specify the features related to the expression of the medical document, such as information indicating a hospital that interprets the target medical image, information indicating a medical department in the hospital, and information indicating a doctor who interprets the image, for example.

In the medical document creation apparatus according to the aspect of the present disclosure, the feature vector may include findings regarding abnormal shadows included in the medical image for learning and the target medical image.

The medical document creation apparatus according to the aspect of the present disclosure may further comprise an analysis unit that analyzes the target medical image to generate the feature vector.

The medical document creation apparatus according to the aspect of the present disclosure may further comprise a display control unit that displays the at least one medical document on a display unit.

In the medical document creation apparatus according to the aspect of the present disclosure, the document creation unit may select a plurality of expression vectors according to the feature information from the plurality of expression vectors and create a plurality of the medical documents, and the display control unit may display the plurality of medical documents on the display unit.

The medical document creation apparatus according to the aspect of the present disclosure may further comprise an input unit that receives an input for selecting a specific medical document from the plurality of medical documents.

The medical document creation apparatus according to the aspect of the present disclosure may further comprise an expression vector selection unit that receives a selection of at least one other expression vector other than the expression vector used in creating the medical document, in which the document creation unit may generate the medical document by using the other expression vector as said selected at least one expression vector, and the display control unit may display the medical document on the display unit by using the other expression vector.

In another aspect, there is provided a learning device according to claim 8 of the appended claims.

The learning device according to the aspect of the present disclosure may further comprise an expression vector generation unit that generates an expression vector of the medical image for learning by using the medical document for learning and the feature vector of the medical image for learning corresponding to the medical document for learning.

In another aspect, there is provided medical document creation method according to claim 10 of the appended claims.

In another aspect, there is provided learning method according to claim 11 of the appended claims.

In another aspect, there is provided computer-readable storage medium according to claim 12 of the appended claims.

In another aspect, there is provided computer-readable storage medium according to claim 13 of the appended claims.

According to the aspects of the present disclosure, it is possible to create a medical document in an expression format according to the features included in the medical image.

### BRIEF DESCRIPTION OF THE DRAWINGS

Fig. 1 is a diagram showing a schematic configuration of a medical information system to which a medical document creation apparatus according to a first embodiment of the present disclosure is applied.
Fig. 2 is a diagram showing a schematic configuration of the medical document creation apparatus according to the first embodiment.
Fig. 3 is a diagram showing items of findings and examples of findings for each item.
Fig. 4 is a diagram showing detection results of findings.
Fig. 5 is a diagram showing a schematic configuration of a document creation unit.
Fig. 6 is a schematic block diagram showing a process performed by an expression vector generation unit.
Fig. 7 is a diagram showing an example of a feature vector of a medical image for learning.
Fig. 8 is a diagram showing an example of a medical document for learning.
Fig. 9 is a diagram showing an interpretation report display screen in the first embodiment.
Fig. 10 is a flowchart showing a medical document creation process performed in the first embodiment.
Fig. 11 is a flowchart showing a learning process performed in the first embodiment.
Fig. 12 is a diagram showing an interpretation report display screen in a second embodiment.
Fig. 13 is a diagram showing an interpretation report display screen in a third embodiment.
Fig. 14 is a diagram showing an expression selection window.

### DEAILED DESCRIPTION

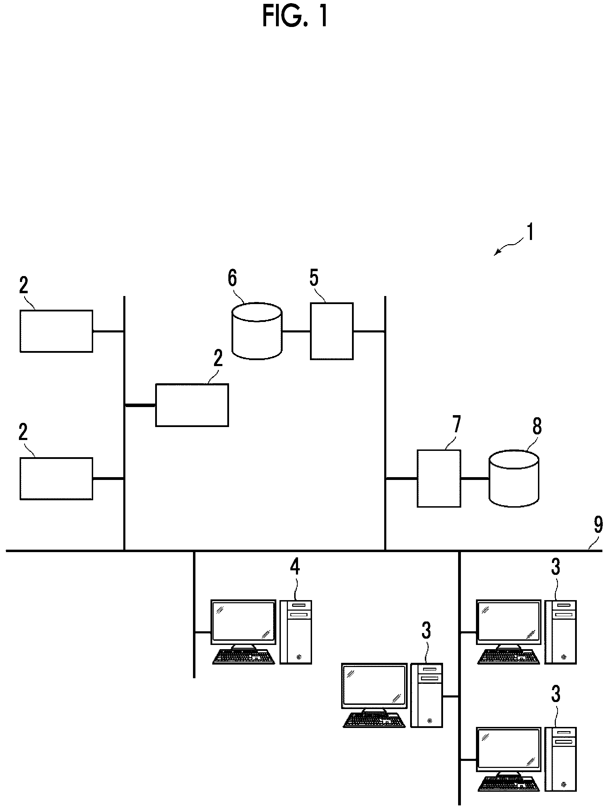

Hereinafter, an embodiment of the present disclosure will be described with reference to the diagrams. Fig. 1 is a diagram showing a schematic configuration of a medical information system to which a medical document creation apparatus according to a first embodiment of the present disclosure is applied. A medical information system 1 shown in Fig. 1 is, based on an examination order from a doctor in a medical department using a known ordering system, a system for imaging an examination target part of a subject, storing a medical image acquired by the imaging, interpreting the medical image by a radiologist and creating an interpretation report, and viewing the interpretation report and observing the medical image to be interpreted in detail by the doctor in the medical department that is a request source. As shown in Fig. 1, the medical information system 1 is configured to include a plurality of modalities (imaging apparatuses) 2, a plurality of interpretation workstations (WS) 3 that are interpretation terminals, a medical department workstation (WS) 4, an image server 5, an image database 6, an interpretation report server 7, and an interpretation report database 8 that are communicably connected to each other through a wired or wireless network 9.

Each apparatus is a computer on which an application program for causing each apparatus to function as a component of the medical information system 1 is installed. The application program is stored in a storage apparatus of a server computer connected to the network 9 or in a network storage in a state in which it can be accessed from the outside, and is downloaded and installed on the computer in response to a request. Alternatively, the application program is recorded on a recording medium, such as a digital versatile disc (DVD) or a compact disc read only memory (CD-ROM), and distributed, and installed on the computer from the recording medium.

The modality 2 is an apparatus that generates a medical image showing a diagnosis target part of the subject by imaging the diagnosis target part. Specifically, examples of the modality include a simple X-ray imaging apparatus, a CT apparatus, an MRI apparatus, a positron emission tomography (PET) apparatus, and the like. A medical image generated by the modality 2 is transmitted to the image server 5 and saved therein.

The interpretation WS 3 encompasses the medical document creation apparatus according to the present embodiment. The configuration of the interpretation WS 3 will be described later.

The medical department WS 4 is a computer used by a doctor in a medical department to observe an image in detail, view an interpretation report, create an electronic medical record, and the like, and is configured to include a processing apparatus, a display apparatus such as a display, and an input apparatus such as a keyboard and a mouse. In the medical department WS 4, each process such as creating a medical record (electronic medical record) of a patient, requesting the image server 5 to view an image, displaying an image received from the image server 5, automatically detecting or highlighting a lesion-like portion in the image, requesting the interpretation report server 7 to view an interpretation report, and displaying the interpretation report received from the interpretation report server 7 is performed by executing a software program for each process.

The image server 5 is a general-purpose computer on which a software program that provides a function of a database management system (DBMS) is installed. The image server 5 comprises a storage in which the image database 6 is configured. This storage may be a hard disk apparatus connected to the image server 5 by a data bus, or may be a disk apparatus connected to a storage area network (SAN) or a network attached storage (NAS) connected to the network 9. In a case where the image server 5 receives a request to register a medical image from the modality 2, the image server 5 prepares the medical image in a format for a database and registers the medical image in the image database 6.

Image data of the medical image acquired by the modality 2 and accessory information are registered in the image database 6. The accessory information includes, for example, an image identification (ID) for identifying each medical image, a patient ID for identifying a subject, an examination ID for identifying an examination, a unique ID (UID: unique identification) allocated for each medical image, examination date and examination time at which a medical image is generated, the type of modality used in an examination for acquiring a medical image, patient information such as the name, age, and gender of a patient, an examination part (imaging part), imaging information (an imaging protocol, an imaging sequence, an imaging method, imaging conditions, the use of a contrast medium, and the like), and information such as a series number or a collection number when a plurality of medical images are acquired in one examination.

In addition, in a case where a viewing request from the interpretation WS 3 is received through the network 9, the image server 5 searches for a medical image registered in the image database 6 and transmits the searched medical image to the interpretation WS 3 that is a request source.

The interpretation report server 7 incorporates a software program for providing a function of a database management system to a computer. In a case where the interpretation report server 7 receives a request to register an interpretation report from the interpretation WS 3, the interpretation report server 7 prepares the interpretation report in a format for a database and registers the interpretation report in the interpretation report database 8. Further, in a case where the request to search for the interpretation report is received, the interpretation report is searched from the interpretation report database 8.

In the interpretation report database 8, for example, an interpretation report is registered in which information, such as an image ID for identifying a medical image to be interpreted, a radiologist ID for identifying an image diagnostician who performed the interpretation, a lesion name, position information of a lesion, findings, and confidence of the findings, is recorded.

The network 9 is a wired or wireless local area network that connects various apparatuses in a hospital to each other. In a case where the interpretation WS 3 is installed in another hospital or clinic, the network 9 may be configured to connect local area networks of respective hospitals through the Internet or a dedicated line.

Hereinafter, the interpretation WS 3 according to the present embodiment will be described in detail. The interpretation WS 3 is a computer used by a radiologist of a medical image to interpret the medical image and create an interpretation report, and is configured to include a processing apparatus, a display apparatus such as a display, and an input apparatus such as a keyboard and a mouse. In the interpretation WS 3, each process such as requesting the image server 5 to view a medical image, various kinds of image processing on the medical image received from the image server 5, displaying the medical image, an analysis process on the medical image, highlighting the medical image based on the analysis result, creating the interpretation report based on the analysis result, supporting the creation of an interpretation report, requesting the interpretation report server 7 to register and view the interpretation report, and displaying the interpretation report received from the interpretation report server 7 is performed by executing a software program for each process. Note that, in these processes, processes other than those performed by the medical document creation apparatus according to the present embodiment are performed by a well-known software program, and therefore the detailed description thereof will be omitted here. In addition, processes other than the processes performed by the medical document creation apparatus according to the present embodiment may not be performed in the interpretation WS 3, and a computer that performs the processes may be separately connected to the network 9, and in response to a processing request from the interpretation WS 3, the requested process may be performed by the computer.

The interpretation WS 3 encompasses the medical document creation apparatus according to the first embodiment in order to create an interpretation report. Further, the medical document creation apparatus according to the first embodiment encompasses a learning device according to the first embodiment. Therefore, a medical document creation program and a learning program according to the first embodiment are installed on the interpretation WS 3. The medical document creation program and the learning program are stored in the storage apparatus of the server computer connected to the network or in the network storage in a state in which it can be accessed from the outside, and are downloaded and installed on the interpretation WS 3 in response to a request. Alternatively, the medical document creation program is recorded on a recording medium such as a DVD or a CD-ROM, distributed, and installed on the interpretation WS 3 from the recording medium.

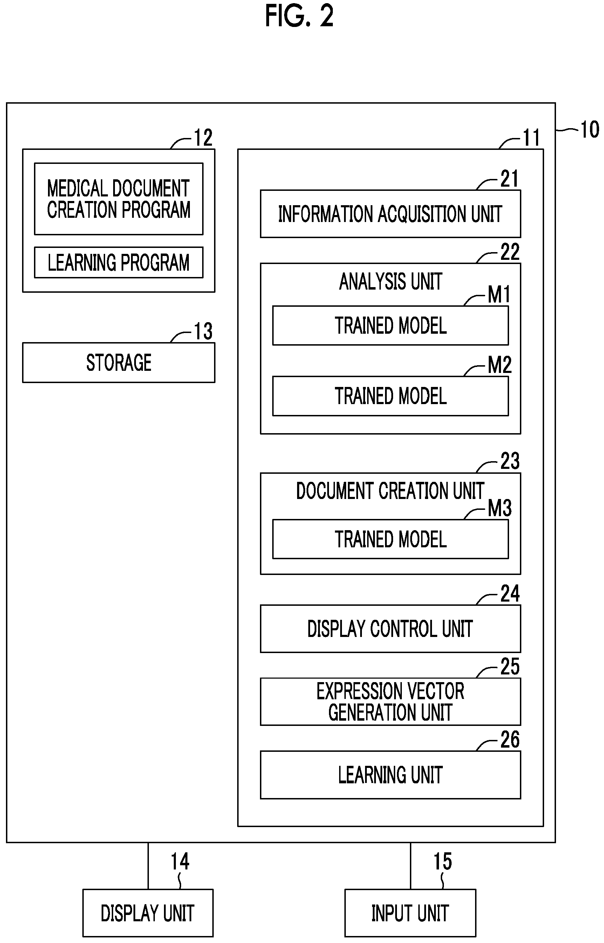

Fig. 2 is a diagram showing a schematic configuration of the medical document creation apparatus according to the first embodiment of the present disclosure, which is realized by installing the medical document creation program and the learning program. As shown in Fig. 2, a medical document creation apparatus 10 comprises a central processing unit (CPU) 11, a memory 12, and a storage 13 as the configuration of a standard computer. A display apparatus (hereinafter, referred to as a display unit) 14, such as a liquid crystal display, and an input apparatus (hereinafter, referred to as an input unit) 15, such as a keyboard and a mouse, are connected to the medical document creation apparatus 10.

The storage 13 consists of a storage device, such as a hard disk or a solid state drive (SSD). The storage 13 stores various kinds of information including medical images and information necessary for processing of the medical document creation apparatus 10, which are acquired from the image server 5 through the network 9. In addition, a plurality of expression vectors, which will be described later, are also stored in the storage 13. The storage 13 corresponds to a storage unit.

Further, the memory 12 stores the medical document creation program and the learning program. The medical document creation program defines, as processes to be executed by the CPU 11, an information acquisition process of acquiring a target medical image for which an interpretation report is to be created and learning data to be described later, an analysis process of analyzing the target medical image to generate a feature vector of the target medical image, a document creation process of creating at least one medical document including features related to the target medical image, and a display control process of displaying the created at least one medical document on a display unit 14. In the present embodiment, it is assumed that the medical document creation apparatus 10 creates an interpretation report as a medical document.

The learning program defines an expression vector generation process of generating an expression vector of the medical image for learning by using the medical document for learning and the feature vector of the medical image for learning corresponding to the medical document for learning, and a learning process of generating a trained model to be described later.

The computer functions as an information acquisition unit 21, an analysis unit 22, a document creation unit 23, a display control unit 24, an expression vector generation unit 25, and a learning unit 26 by the CPU 11 executing these processes according to the medical document creation program.

In the present embodiment, it is assumed that a CT image including the lungs of the human body is used as a target medical image, and an interpretation report on lung nodules is created.

The information acquisition unit 21 acquires a target medical image G0 to be created for the interpretation report from the image server 5 through an interface (not shown) connected to the network 9. In addition, learning data and the like used in generating a trained model, which will be described later, are also acquired. In a case where the target medical image G0 and the learning data are already stored in the storage 13, the information acquisition unit 21 may acquire the target medical image G0 and the learning data from the storage 13.

The analysis unit 22 analyzes the target medical image G0 to detect abnormal shadows such as lesions, and generates a feature vector related to the abnormal shadows. For this purpose, the analysis unit 22 has a trained model M1 for detecting an abnormal shadow from the target medical image G0 and a trained model M2 for generating a feature vector. In the present embodiment, the trained model M1 consists of, for example, a convolutional neural network (CNN) for which deep learning has been performed so as to determine whether or not each pixel in the target medical image G0 has an abnormal shadow. The trained model M1 is generated by learning the CNN so as to output a determination result of whether or not each pixel in the target region in the target medical image G0 has a pixel value that can be regarded as an abnormal shadow in a case where the target medical image G0 is input. The trained model M1 derives an output value, for example, a value of 0 to 1, representing the probability of various findings that can be regarded as abnormal shadows for each pixel of the target medical image G0, and outputs a determination result of determining a pixel whose output value is equal to or higher than a predetermined threshold value as an abnormal shadow. In learning the CNN for generating the trained model M1, a large number of medical images whose determination results of abnormal shadows for each pixel are known are used as learning data.

The trained model M2 detects a plurality of findings representing features related to abnormal shadows based on the determination result output by the trained model M1. In the present embodiment, in a case where the determination result of the region including the abnormal shadow in the target medical image G0 output by the trained model M1 is input, the trained model M2 consists of, for example, a CNN for which deep learning has been performed so as to output the detection results of findings for items of a plurality of types of findings related to abnormal shadows in that region. In learning the CNN for generating the trained model M2, a large number of medical images whose detection results of findings for abnormal shadows are known are used as learning data.

Further, in a case where the pixels determined to be abnormal shadows are grouped together to exist as a region, the trained model M2 is also trained to output the size of the region and the position of the region in the lungs. The size is the vertical and horizontal size or diameter of the region represented in units such as mm or cm. The position is represented by, for example, the left and right lung areas S1 to S10 or the left and right lung lobes (upper lobe, middle lobe, and lower lobe) where the centroid position of the region exists.

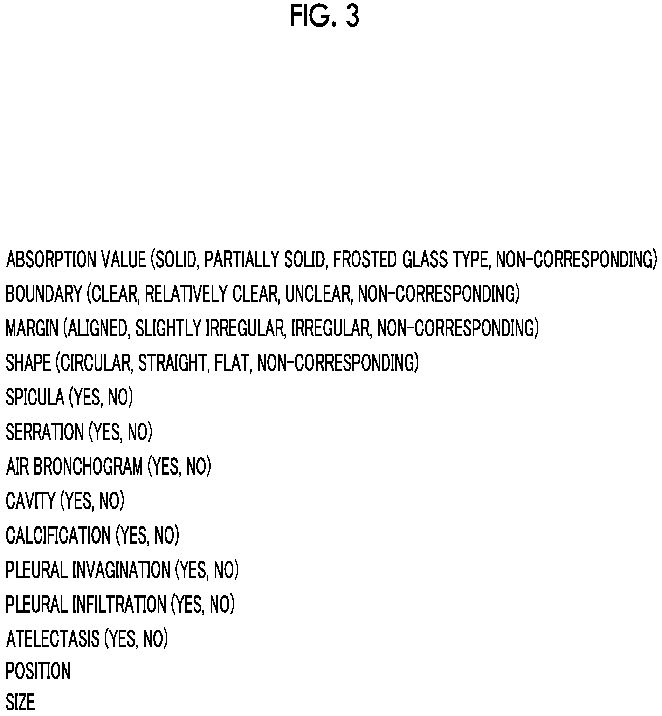

Fig. 3 is a diagram showing items of findings and examples of findings for each item. In the present embodiment, the target medical image G0 is a CT image of the lung, and the abnormal shadow is a candidate for a lung nodule. Therefore, Fig. 3 shows the items of findings regarding lung nodules. Further, in Fig. 3, findings for items are shown in parentheses corresponding to the items of the findings. As shown in Fig. 3, the items of the findings and the findings for the items include an absorption value (solid, partially solid, frosted glass type, non-corresponding), a boundary (clear, relatively clear, unclear, non-corresponding), a margin (aligned, slightly irregular, irregular, non-corresponding), a shape (circular, straight, flat, non-corresponding), spicula (yes, no), serration (yes, no), an air bronchogram (yes, no), a cavity (yes, no), calcification (yes, no), a pleural invagination (yes, no), a pleural infiltration (yes, no), atelectasis (yes, no), a position, and a size.

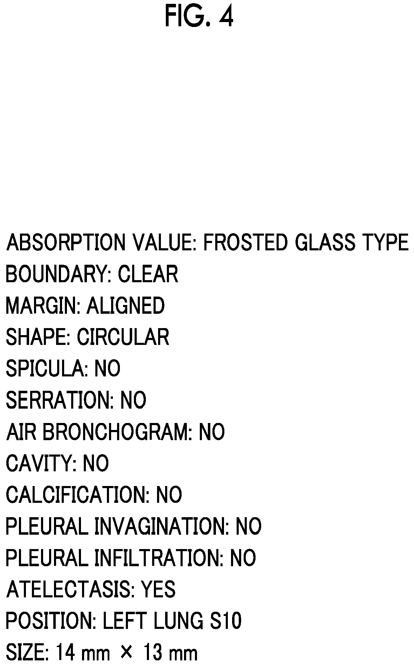

In the present embodiment, the trained model M2 of the analysis unit 22 detects findings for all the above items and outputs detection results. Fig. 4 is a diagram showing detection results of findings. The detection results of the findings shown in Fig. 4 show that the absorption value: frosted glass type, the boundary: clear, the margin: aligned, the shape: circular, the spicula: no, the serration: no, the air bronchogram: no, the cavity: no, the calcification: no, the pleural invagination: no, the pleural infiltration: no, the atelectasis: yes, the position: left lung S10, and the size: 14 mm × 13 mm.

The analysis unit 22 generates a feature vector representing the findings output by the trained model M2, and outputs the feature vector as an analysis result. Here, it is assumed that the feature vector representing the findings has elements of x1 to x14 corresponding to the item of the finding. Elements x1 to x14 correspond to an absorption value, a boundary, a margin, a shape, spicula, serration, air bronchogram, a cavity, a calcification, pleural invagination, pleural infiltration, atelectasis, a position, and a size, respectively. Each of the elements x1 to x14 has a parameter corresponding to the finding. The parameters take values according to the findings. For example, the parameter of the element x1 corresponding to the absorption value takes a value of 0 to 3 for each of the solid type, the partially solid type, the frosted glass type, and the non-corresponding type. The parameter of the element x2 corresponding to the boundary takes a value of 0 to 3 for each of clear, relatively clear, unclear, and non-corresponding. The parameter of the element x3 corresponding to the margin takes a value of 0 to 3 for each of aligned, slightly irregular, irregular, and non-corresponding. The parameter of the element x4 corresponding to the shape takes a value of 0 to 3 for each of circular, straight, flat, and non-corresponding. The parameter of the element x5 corresponding to the spicula takes a value of 0 and 1 for each of yes and no. The parameter of the element x6 corresponding to the serration takes a value of 0 and 1 for each of yes and no. The parameter of the element x7 corresponding to the air bronchogram takes a value of 0 and 1 for each of yes and no. The parameter of the element x8 corresponding to the cavity takes a value of 0 and 1 for each of yes and no. The parameter of the element x9 corresponding to the calcification takes a value of 0 and 1 for each of yes and no. The parameter of the element x10 corresponding to the pleural invagination takes a value of 0 and 1 for each of yes and no. The parameter of the element x11 corresponding to the pleural infiltration takes a value of 0 and 1 for each of yes and no. The parameter of the element x12 corresponding to the atelectasis takes a value of 0 and 1 for each of yes and no. The parameter of the element x13 corresponding to the position takes a value of 0 to 9 for each of the left lung lobes S1 to S10 and a value of 10 to 18 for each of the right lung lobes S1 to S10. The parameter of the element x14 corresponding to the size takes a value of the size.

For example, as shown in Fig. 4, in the case of the absorption value: frosted glass type, the boundary: clear, the margin: aligned, the shape: circular, the spicula: no, the serration: no, the air bronchogram: no, the cavity: no, the calcification: no, the pleural invagination: no, the pleural infiltration: no, the atelectasis: yes, the position: left lung S10, and the size: 14 mm × 13 mm, a feature vector C0 is represented by (x1, x2, x3, x4, x5, x6, x7, x8, x9, x10, x11, x12, x13, x14) = (2,0,0,0,1,1,1,1,1,1,1,0,9,14 × 13).

The document creation unit 23 creates at least one interpretation report including features related to the target medical image G0 as a medical document. For this purpose, the document creation unit 23 has a trained model M3 that selects at least one expression vector according to the feature vector of the target medical image G0 from the plurality of expression vectors in a case where the feature vector of the target medical image G0 is input, and that creates at least one interpretation report including features related to the target medical image G0 in a case where the feature vector of the target medical image G0 and the selected at least one expression vector are input.

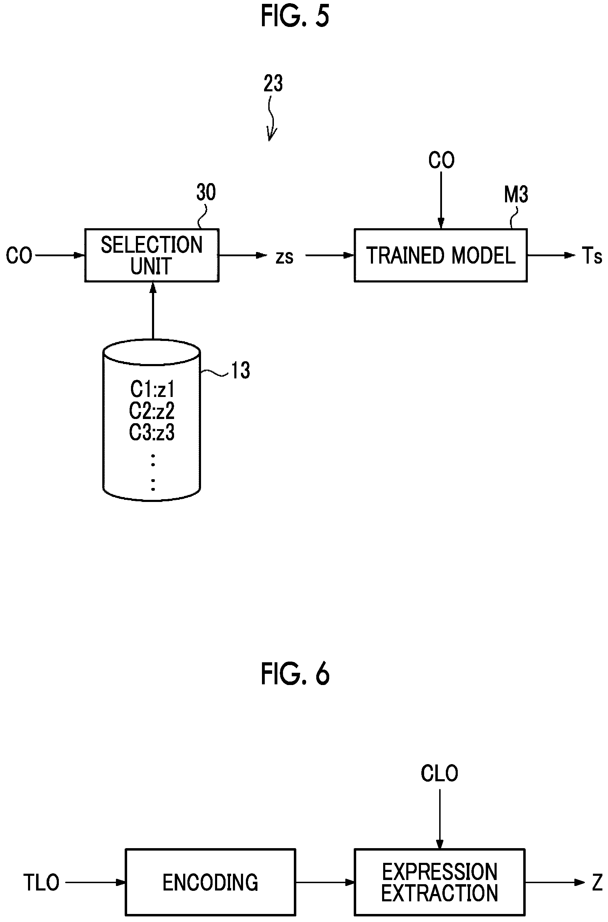

Fig. 5 is a diagram showing a schematic configuration of the document creation unit 23. As shown in Fig. 5, the document creation unit 23 includes a selection unit 30 and a trained model M3. The selection unit 30 selects at least one expression vector zs according to the feature vector C0 of the target medical image G0 input to the document creation unit 23 from a plurality of expression vectors zi (i = 2 to n: n is the number of expression vectors) saved in the storage 13. In the first embodiment, it is assumed that one expression vector zs is selected. The plurality of expression vectors zi are generated by the expression vector generation unit 25 and saved in the storage 13 in a case where the learning unit 26 learns the CNN as described later in order to generate the trained model M3. The generation of the expression vector will be described below.

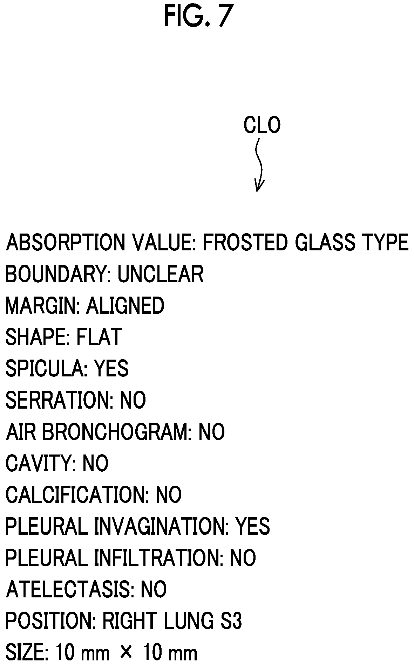

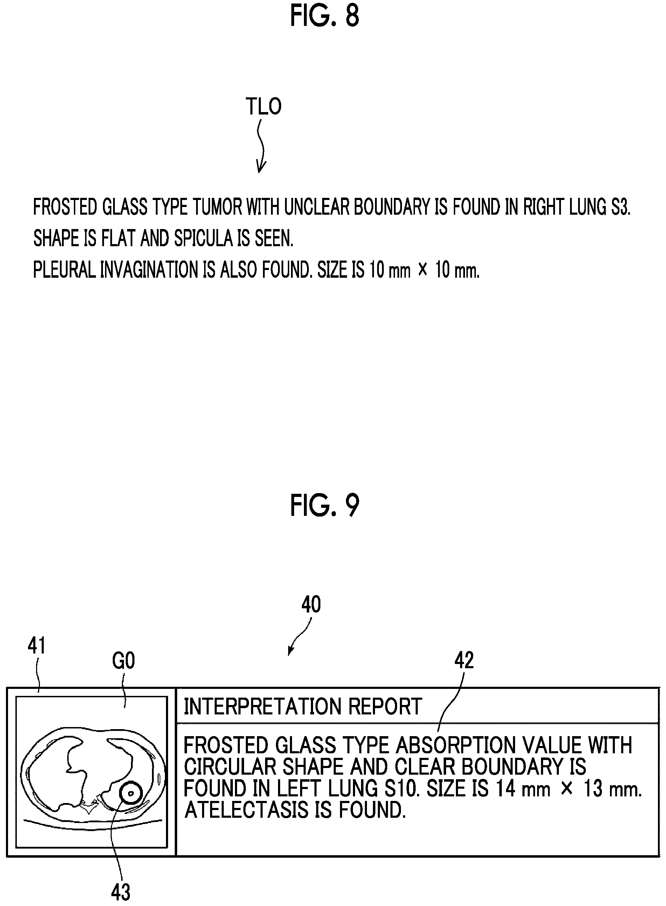

Fig. 6 is a schematic block diagram showing a process performed by the expression vector generation unit. In generating the expression vector, a feature vector CL0 of a medical image GL0 for learning so as to learn the trained model M3 of the document creation unit 23 and a medical document TL0 for learning related to the medical image GL0 for learning are used as described later. Fig. 7 is a diagram showing an example of a feature vector of a medical image for learning, and Fig. 8 is a diagram showing an example of a medical document for learning. In Fig. 7, the feature vector CL0 is shown by the findings themselves. The feature vector CL0 of the medical image GL0 for learning shows that the absorption value: frosted glass type, the boundary: unclear, the margin: aligned, the shape: flat, the spicula: yes, the serration: no, the air bronchogram: no, the cavity: no, the calcification: no, the pleural invagination: yes, the pleural infiltration: no, the atelectasis: no, the position: right lung S3, and the size: 10 mm × 10 mm. The feature vector CL0 of the medical image GL0 for learning may be generated by analyzing the medical image GL0 for learning by the analysis unit 22.

Further, the medical document for learning TL0 shown in Fig. 8 was created in advance by a radiologist based on the feature vector C0 (that is, findings) of the medical image GL0 for learning, and the content thereof is "A frosted glass type tumor with an unclear boundary is found in the right lung S3. The shape is flat and spicula is seen. Pleural invagination is also found. The size is 10 mm × 10 mm."

In a case where the feature vector CL0 of the medical image for learning and the medical document TL0 for learning are input, the expression vector generation unit 25 encodes the medical document TL0 for learning. Then, the expression vector generation unit 25 generates an expression vector z representing the expression of the medical document TL0 for learning by removing the component related to the feature vector CL0 from the encoded medical document TL0 for learning. For example, the expression vector z includes elements such as whether the sentence is active or passive, whether the ending used is a definite tone (is, are, etc.) or an estimated tone (believed to be ..., looks like ..., ... is found, etc.), the length of the sentence, the order in which the features are mentioned in the sentence, and the words used for the same finding (for example, "frosted glass nodules" or "localized frosted glass shadows" for the frosted glass type).

The elements of the expression vector z are represented by h1, h2, h3, and the like. The elements h1, h2, h3, and the like each represent whether the sentence is active or passive, whether the ending used is a definite tone or an estimated tone, the length of the sentence, and the like. The elements h1, h2, h3, and the like each have a parameter that takes a value according to the content of the element. For example, the parameter of the element h1 corresponding to whether the sentence is active or passive takes a value of 0 and 1 for each of the active form and the passive form. Further, the parameter of the element h2 corresponding to whether the ending used is a definite tone or an estimated tone takes a value of 0 and 1 for each of the definite tone or the estimated tone.

In the present embodiment, the expression vector generation unit 25 generates a plurality of expression vectors zi using a set of a plurality of medical images for learning and a medical document for learning. The plurality of generated expression vectors zi are associated with the feature vector that generated the expression vector zi, and are saved in the storage 13.

In selecting the expression vector zs from the plurality of expression vectors zi, the selection unit 30 derives the similarity between the feature vector C0 of the target medical image G0 and a feature vector Ci associated with the expression vector zi. The similarity may be a distance between the feature vector C0 and the plurality of feature vectors Ci. In this case, the smaller the distance, the greater the similarity. Then, the selection unit 30 selects the expression vector zs associated with the feature vector having the highest similarity from the plurality of expression vectors zi.

The trained model M3 consists of, for example, a CNN for which deep learning has been performed so as to output an interpretation report including features related to the target medical image G0 in a case where the selected expression vector zs is input to the target medical image G0 and the target medical image G0. The CNN for generating the trained model M3 is trained by the learning unit 26. The CNN before learning corresponds to a learning model.

The learning unit 26 learns CNN by using a plurality of feature vectors CL0 of the medical image GL0 for learning and a plurality of medical documents TL0 for learning, which are related to the medical image GL0 for learning, as learning data. Specifically, in a case where the feature vector CL0 shown in Fig. 7 and the medical document TL0 for learning shown in Fig. 8 are input, the learning unit 26 causes the expression vector generation unit 25 to generate an expression vector zL0 of the medical document TL0 for learning. Then, in a case where the feature vector CL0 of the medical image GL0 for learning and the expression vector zL0 for learning are input, the learning unit learns the CNN so as to output the medical document TL0 for learning shown in Fig. 8 to generate the trained model M3.

The document creation unit 23 in the present embodiment comprises the selection unit 30 and the trained model M3 as described above. Therefore, in a case where the feature vector C0 of the target medical image G0 is as shown in Fig. 4, the selection unit 30 selects the expression vector zs associated with the feature vector similar to the feature vector C0 from the plurality of expression vectors zi. The feature vector C0 and the selected expression vector zs are input to the trained model M3. In a case where the feature vector C0 and the expression vector zs are input, the trained model M3 outputs an interpretation report including features related to the target medical image G0.

The display control unit 24 displays a interpretation report display screen for displaying the interpretation report created by the document creation unit 23 on the display unit 14. Fig. 9 is a diagram showing an interpretation report display screen in the first embodiment. As shown in Fig. 9, an interpretation report display screen 40 has a display region 41 of the target medical image G0, and a creation region 42 for inputting to create an interpretation report. In addition, an interpretation report of "A frosted glass type absorption value with a circular shape and a clear boundary is found in the left lung S10. The size is 14 mm × 13 mm. Atelectasis is found." created by the document creation unit 23 based on the feature vector C0 shown in Fig. 4 is inserted in the creation region 42. Further, in the target medical image G0 displayed in the display region 41, a circular mark 43 is given at the position of the abnormal shadow. An operator can check the contents of the interpretation report displayed in the creation region 42, and can correct the interpretation report by using the input unit 15 as necessary.

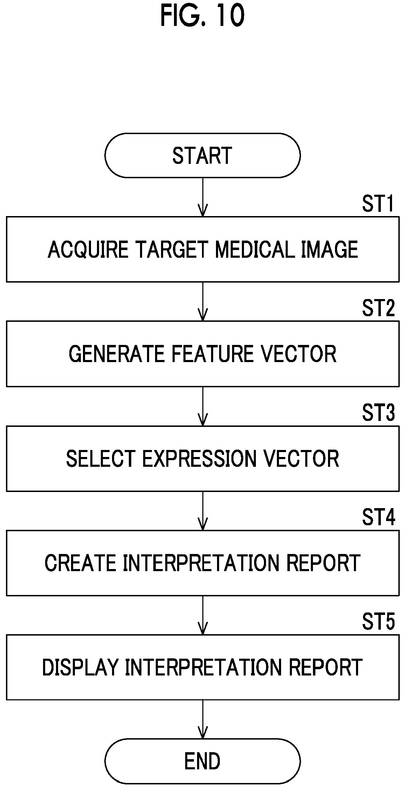

Next, a process performed in the first embodiment will be described. Fig. 10 is a flowchart showing a medical document creation process performed in the first embodiment. The process is started in a case where the operator gives an instruction to create the interpretation report, and the information acquisition unit 21 acquires the target medical image G0 to be created for the interpretation report (step ST1). Next, the analysis unit 22 analyzes the target medical image G0 and generates the feature vector C0 of the target medical image G0 (step ST2). Next, the selection unit 30 of the document creation unit 23 selects the expression vector zs according to the feature vector C0 from the plurality of expression vectors zi stored in the storage 13 (step ST3). Then, the feature vector C0 and the expression vector zs are input to the trained model M3 of the document creation unit 23, and an interpretation report including the features of the target medical image G0 is created (step ST4). Then, the display control unit 24 displays the created interpretation report on the display unit 14 (step ST5), and the process ends.

As necessary, the operator corrects the created interpretation report by inputting from the input unit 15. After that, in a case where the operator instructs the interpretation WS 3 to transmit the interpretation report to the interpretation report server 7, the interpretation report is transmitted to the interpretation report server 7 in association with the target medical image G0 and saved.

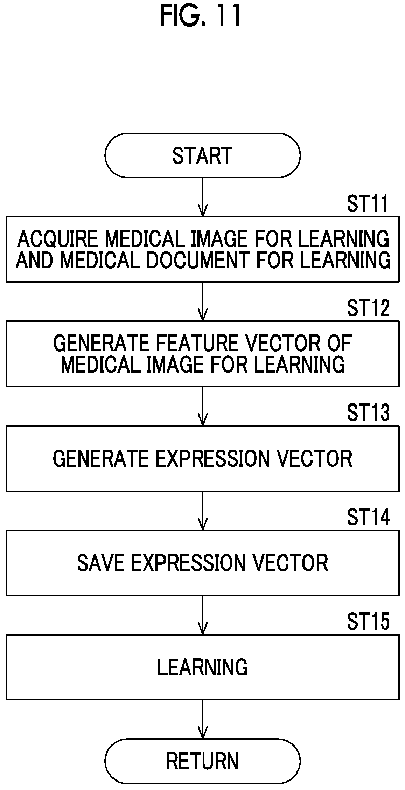

Fig. 11 is a flowchart showing a learning process performed in the present embodiment. First, the information acquisition unit 21 acquires the medical image GL0 for learning and the medical document TL0 for learning created for the medical image GL0 for learning (step ST11). Next, the analysis unit 22 analyzes the medical image GL0 for learning and generates the feature vector CL0 of the medical image GL0 for learning (step ST12). Then, the expression vector generation unit 25 generates the expression vector zL0 from the feature vector CL0 of the medical image GL0 for learning and the medical document TL0 for learning (step ST13), and associates the feature vector CL0 with the expression vector zL0 to save the feature vector CL0 and the expression vector zL0 in the storage 13 (step ST14).

Further, in a case where the feature vector CL0 of the medical image GL0 for learning and the expression vector zL0 are input, the learning unit 26 learns the CNN so as to output the medical document TL0 for learning (step ST15), and returns to step ST11. The learning unit 26 repeats the processes from step ST11 to step ST15 for a plurality of pieces of learning data. Thereby, a plurality of expression vectors zi are stored in the storage 13, and the trained model M3 is generated.

In this way, in the present embodiment, in the case where the feature vector C0 of the target medical image G0 is input, the expression vector zs corresponding to the feature vector C0 of the target medical image G0 is selected from the plurality of expression vectors zi, and in the case where the feature vector C0 of the target medical image G0 and the selected expression vector zs are input to the trained model M3 of the document creation unit 23, an interpretation report including features related to the target medical image G0 is created. Here, the expression vector zs represents an expression format according to the feature vector C0 of the target medical image G0. Therefore, the created interpretation report includes features related to the target medical image G0 and has an expression format according to the features. Therefore, according to the present embodiment, it is possible to create an interpretation report in an expression format according to the features included in the target medical image G0.

Next, a second embodiment of the present disclosure will be described. Since the configuration of a medical document creation apparatus according to the second embodiment is the same as the configuration of the medical document creation apparatus 10 according to the first embodiment shown in Fig. 2 and only the processing to be performed is different, detailed description of the apparatus will be omitted here.

In the first embodiment, the selection unit 30 of the document creation unit 23 selects one expression vector zs according to the feature vector C0 of the target medical image G0 to create one interpretation report. The second embodiment is different from the first embodiment in that the selection unit 30 of the document creation unit 23 selects a plurality of expression vectors zsk (k = 2 to m, m is a positive integer) according to the feature vector C0 of the target medical image G0, and the document creation unit 23 creates a plurality of interpretation reports by using the plurality of selected expression vectors zsk.

In the second embodiment, the selection unit 30 selects a plurality of expression vectors zsk associated with a plurality of feature vectors Ck similar to the feature vector C0. Specifically, a plurality of expression vectors zsk associated with a plurality of feature vectors Ck whose distances from the feature vector C0 are within a predetermined range are selected. In a case where there is only one feature vector whose distance from the feature vector C0 is within a predetermined range, the range of the distance is expanded to select a plurality of expression vectors zsk.

In the second embodiment, the document creation unit 23 inputs the feature vector C0 of the target medical image G0 and each of the plurality of selected expression vectors zsk into the trained model M3, and creates a plurality of interpretation reports by outputting the interpretation reports corresponding to each of the plurality of expression vectors zsk from the trained model M3. The display control unit 24 displays the plurality of interpretation reports created by the document creation unit 23 on the display unit 14.

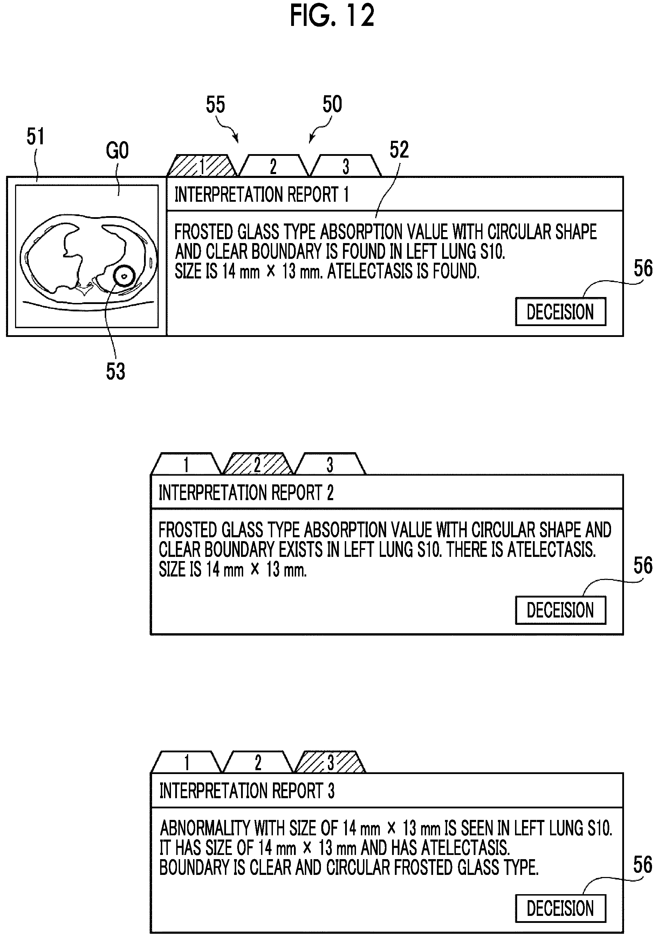

Fig. 12 is a diagram showing an interpretation report display screen in the second embodiment. In the second embodiment, it is assumed that the three expression vectors zs1 to zs3 are selected and three interpretation reports are created. As shown in Fig. 12, an interpretation report display screen 50 has a display region 51 of the target medical image G0, and a creation region 52 for inputting to create an interpretation report. In the target medical image G0 displayed in the display region 51, a circular mark 53 is given at the position of the abnormal shadow. On the upper side of the creation region 52, a tab 55 for switching between the three interpretation reports is displayed. In the initial state, the tab of "1" is selected, and as an interpretation report 1, the interpretation report of "A frosted glass type absorption value with a circular shape and a clear boundary is found in the left lung S10. The size is 14 mm × 13 mm. Atelectasis is found." is displayed in the creation region 52.

In a case where the operator switches the tab 55 to 2, an interpretation report 2 of "A frosted glass type absorption value with a circular shape and a clear boundary exists in the left lung S10. There is atelectasis. The size is 14 mm × 13 mm" is displayed in the creation region 52.

In a case where the operator switches the tab 55 to 3, an interpretation report 3 of "An abnormality with a size of 14 mm × 13 mm is seen in the left lung S10. It has a size of 14 mm × 13 mm and has atelectasis. The boundary is clear and circular frosted glass type." is displayed in the creation region 52.

In the second embodiment, a decision button 56 is displayed in the creation region 52. The operator selects the decision button 56 using the input unit 15 in a state where the desired interpretation report among the three interpretation reports is displayed. Thereby, the selected interpretation report is associated with the target medical image G0, transmitted to the interpretation report server 7, and saved.

In the above second embodiment, although a plurality of interpretation reports are switched and displayed, the present disclosure is not limited thereto. A plurality of interpretation reports may be displayed at once.

Next, a third embodiment of the present disclosure will be described. Since the configuration of a medical document creation apparatus according to the third embodiment is the same as the configuration of the medical document creation apparatus 10 according to the first embodiment shown in Fig. 2 and only the processing to be performed is different, detailed description of the apparatus will be omitted here.

The third embodiment is different from the first embodiment in that expression vectors can be selected, which is different from the case where the displayed interpretation report is created on the interpretation report display screen in the first embodiment.

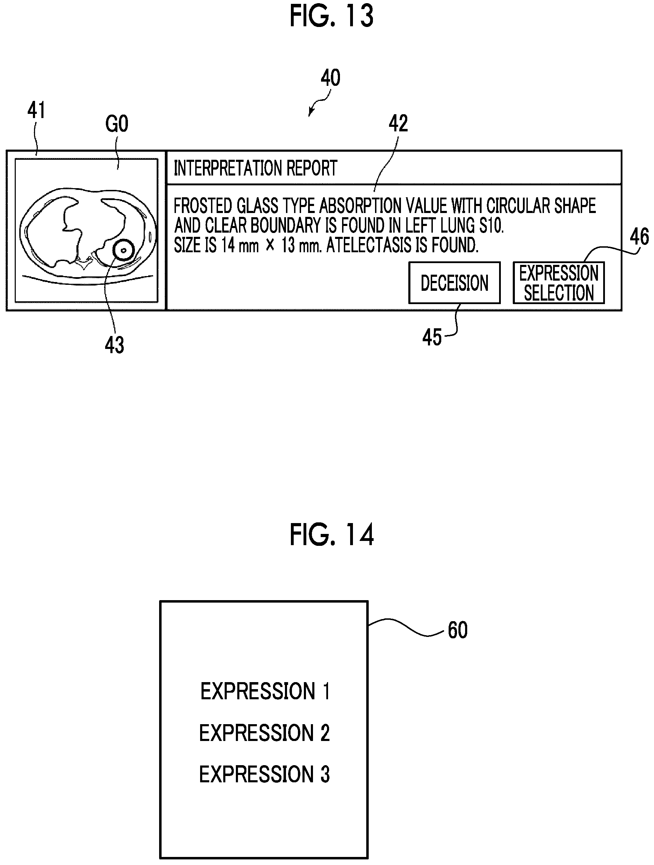

Fig. 13 is a diagram showing an interpretation report display screen in the third embodiment. As shown in Fig. 13, the interpretation report display screen 40 is the same as the interpretation report display screen in the first embodiment, but a decision button 45 and an expression selection button 46 are further displayed. In a case where the operator selects the expression selection button 46 using the input unit 15, the display control unit 24 displays an expression selection window 60 shown in Fig. 14 on the display unit 14. Expressions 1 to 3 are displayed in the expression selection window 60. The expressions 1 to 3 correspond to expression vectors associated with three feature vectors whose distances from the feature vector C0 of the target medical image G0 are in a predetermined range. It is assumed that the distance from the feature vector C0 increases in the order of expressions 1, 2, and 3, that is, the similarity decreases. Further, in the third embodiment, although the interpretation report using the expression vector (that is, expression 1) most similar to the feature vector C0 is displayed in the initial creation region 42 of the interpretation report display screen 40, the present disclosure is not limited thereto.

The operator can select the expression selection button 46 and select a desired expression from the expression selection window 60 displayed thereby. In a case where the operator selects the desired expression, the document creation unit 23 inputs the expression vector corresponding to the selected expression and the feature vector C0 of the target medical image G0 into the trained model M3, and creates an interpretation report of the expression corresponding to the selected expression vector. The display control unit 24 displays the created interpretation report on the interpretation report display screen 40.

According to the third embodiment, the operator can check the interpretation report of different expressions for the target medical image G0 having the same findings.

In the third embodiment, although three expression vectors can be selected in the expression selection window 60, the present disclosure is not limited thereto. In addition to the expression vector associated with the feature vector most similar to the feature vector C0, any number of expression vectors may be selected as long as one or more expression vectors can be selected.

In the above embodiments, although the analysis unit 22 of the medical document creation apparatus 10 in the interpretation WS 3 analyzes the target medical image G0, detects an abnormal shadow, and generates a feature vector, the present disclosure is not limited thereto. The analysis process may be performed on an external analysis server (not shown) or the like. In this case, the information acquisition unit 21 may acquire the feature vector acquired by an external analysis server or the like, and the acquired feature vector may be used to create an interpretation report and learn the trained model M3. In this case, the medical document creation apparatus 10 does not need the analysis unit 22.

Further, in the above embodiments, although the analysis unit 22 generates a feature vector representing the findings, the present disclosure is not limited thereto. The output value output by the trained model M1 may be used as the feature vector. In this case, the plurality of expression vectors zi are associated with the feature vectors representing the output values and saved in the storage 13. Further, the expression vector generation unit 25 generates an expression vector based on the medical document TL0 for learning and the feature vector representing the output value. That is, by removing the component of the output value output by the trained model M1 from the encoded medical document TL0 for learning, the parameter representing the expression of the medical document TL0 for learning is extracted and the expression vector is generated.

Further, in the above embodiments, both the feature vector representing the findings and the feature vector based on the output value output by the trained model M1 may be used. In this case, the plurality of expression vectors zi are associated with both the feature vector representing the findings and the feature vector representing the output value, and saved in the storage 13. Further, the expression vector generation unit 25 generates an expression vector based on the medical document TL0 for learning and the feature vectors representing the finding and the output value. That is, by removing the components of the finding and the output value output by the trained model M1 from the encoded medical document TL0 for learning, the parameter representing the expression of the medical document TL0 for learning is extracted and the expression vector is generated.

Further, in the above embodiments, although the expression vector is associated with the feature vector and saved in the storage 13, the present disclosure is not limited thereto. The expression vector may be saved in association with feature information obtained by adding the accessory information of the target medical image to the feature vector. Here, in the interpretation report, the rules of the expression format may be determined according to the hospital. In this case, the expression format of the interpretation report differs depending on the hospital. Further, even within the same hospital, the rules of the expression format for creating the interpretation report may differ depending on the medical department, and in this case, the expression format of the interpretation report differs depending on the medical department. Furthermore, the expression format of the interpretation report differs depending on the doctor. Therefore, as the accessory information, it is possible to use information that can specify the features related to the expression in the case of creating the medical document, such as information indicating a hospital that interprets the target medical image, information indicating a medical department in the hospital, and information indicating a doctor who interprets the image, for example. In this case, the feature information, that is, the expression vector according to both the feature vector and the accessory information is selected from the plurality of expression vectors and used for creating the medical document in the document creation unit 23.

In this way, by selecting the expression vector using the accessory information as well, it is possible to create an interpretation report in an expression format according to the hospital, medical department, doctor, or the like.

Further, in the above embodiments, although the medical document creation apparatus 10 comprises the expression vector generation unit 25 and the learning unit 26, the present disclosure is not limited thereto. An external learning device (not shown) may learn the CNN to generate the trained model M3. In this case, the trained model M3 generated by the external learning device is installed in the medical document creation apparatus 10. Further, in this case, the medical document creation apparatus 10 does not need the expression vector generation unit 25 and the learning unit 26.

Further, in the above embodiments, although the interpretation report regarding the lung nodule in the lung is created, the target for creating the interpretation report is not limited to the lung. The technique of the present disclosure can also be applied to a case of creating an interpretation report for a medical image including a diagnostic target other than the lung such as the brain, liver, heart, and kidney. In this case, the trained models M1 and M2 of the analysis unit 22 are trained to generate a feature vector for the medical image to be diagnosed. Further, the trained model M3 of the document creation unit 23 is trained to output an interpretation report according to the feature vector of the diagnosis target.

In addition, in the above embodiments, although the technique of the present disclosure is applied to the case of creating an interpretation report as a medical document, the technique of the present disclosure can also be applied to a case of creating medical documents other than the interpretation report, such as an electronic medical record and a diagnosis report. In this case, the trained model M3 of the document creation unit 23 is trained by using the medical document of the type to be created as the medical document TL0 for learning. Further, in this case, the expression vector according to the medical document to be created is saved in the storage 13.

Further, in the above embodiments, the trained models M1 to M3 are not limited to CNN. In addition to CNN, a support vector machine (SVM), a deep neural network (DNN), a recurrent neural network (RNN), and the like can be used.

Further, in the above embodiments, for example, as hardware structures of processing units that execute various kinds of processing, such as the information acquisition unit 21, the analysis unit 22, the document creation unit 23, the display control unit 24, the expression vector generation unit 25, and the learning unit 26, various processors shown below can be used. As described above, the various processors include a programmable logic device (PLD) as a processor of which the circuit configuration can be changed after manufacture, such as a field programmable gate array (FPGA), a dedicated electrical circuit as a processor having a dedicated circuit configuration for executing specific processing such as an application specific integrated circuit (ASIC), and the like, in addition to the CPU as a general-purpose processor that functions as various processing units by executing software (program).

One processing unit may be configured by one of the various processors, or configured by a combination of the same or different kinds of two or more processors (for example, a combination of a plurality of FPGAs or a combination of the CPU and the FPGA). In addition, a plurality of processing units may be configured by one processor.

As an example where a plurality of processing units are configured by one processor, first, there is a form in which one processor is configured by a combination of one or more CPUs and software as typified by a computer, such as a client or a server, and this processor functions as a plurality of processing units. Second, there is a form in which a processor for realizing the function of the entire system including a plurality of processing units by one integrated circuit (IC) chip as typified by a system on chip (SoC) or the like is used. In this way, various processing units are configured by one or more of the above-described various processors as hardware structures.

Furthermore, as the hardware structure of the various processors, more specifically, an electrical circuit (circuitry) in which circuit elements such as semiconductor elements are combined can be used.

### Explanation of References

1: medical information system
2: modality
3: interpretation workstation
4: medical department workstation
5: image server
6: image database
7: interpretation report server
8: interpretation report database
9: network
10: medical document creation apparatus
11: CPU
12: memory
13: storage
14: display unit
15: input unit
21: information acquisition unit
22: analysis unit
23: document creation unit
24: display control unit
25: expression vector generation unit
26: learning unit
30: selection unit
40, 50: interpretation report display screen
41, 51: display region
42, 52: creation region
43, 53: mark
44, 54: window
45: decision button
46: expression selection button
55: tab
56: decision button
60:expression selection window
C0, Ci: feature vector
CL0: feature vector of medical image for learning
z, zs, zi: expression vector
TL0: medical document for learning
G0: target medical image
M1 to M3: trained model

## Claims

1. A medical document creation apparatus (10) comprising:
a document creation unit (23) having a trained model (M3) that has been trained to output a medical document (Ts) including features related to a target medical image (G0) in a case where a feature vector (C0) of the target medical image (G0) and an expression vector (zs) according to feature information including the feature vector (C0) of the target medical image (G0) are input, wherein the trained model (M3) has been trained by using a plurality of pieces of learning data each including a feature vector (CL0) of a medical image for learning (GL0) and an expression vector (z) of a medical document for learning (TLO), wherein a feature vector (C0,CL0) represents findings, the findings comprising, for each of a plurality of items, a respective finding; and
a storage unit (13) that stores a plurality of expression vectors (z1,z2,z3...) corresponding to feature vectors (CL0) of each of a plurality of the medical images for learning (GL0) , wherein each expression vector (z1,z2,z3...) represents the expression of the medical document for learning (TL0) and is generated by removing a component related to the feature vector (CL0) from the encoded medical document for learning (TL0) after encoding the medical document for learning (TLO),
wherein, in a case where the feature information including the feature vector (C0) of the target medical image (G0) is input, the document creation unit (23) selects at least one expression vector (zs) according to the feature information from the plurality of expression vectors (z1,z2,z3...) stored in the storage unit (13), inputs the feature vector (C0) of the target medical image (G0) and the selected at least one expression vector (zs) into the trained model (M3), and creates at least one medical document (Ts) including features related to the target medical image (G0).

2. The medical document creation apparatus (10) according to claim 1, wherein the feature vector (C0, CL0) includes findings regarding abnormal shadows included in the medical image for learning (GL0) and the target medical image (G0).

3. The medical document creation apparatus (10) according to claim 1 or 2, further comprising: an analysis unit (22) that analyzes the target medical image (G0) to generate the feature vector (C0, CL0).

4. The medical document creation apparatus (10) according to any one of claims 1 to 3, further comprising:
a display control unit (24) that displays the at least one medical document (Ts) on a display unit (14).

5. The medical document creation apparatus (10) according to claim 4, wherein
the document creation unit (23) selects a plurality of expression vectors (z, zs, zi) according to the feature information of the target medical image (G0) from the stored plurality of expression vectors ( z1,z2,z3... ) and creates a plurality of the medical documents (Ts), and
the display control unit (24) displays the plurality of medical documents (Ts) on the display unit (14).

6. The medical document creation apparatus (10) according to claim 5, further comprising: an input unit (15) that receives an input for selecting a specific medical document (Ts) from the plurality of medical documents (Ts).

7. The medical document creation apparatus (10) according to claim 4 or 5, further comprising:
an expression vector selection unit (30) that receives a selection of at least one other expression vector other than the expression vector (z, zs, zi) used in creating the medical document (Ts), wherein
the document creation unit (23) generates the medical document (Ts) by using the other expression vector as said selected at least one expression vector (zs), and
the display control unit (24) displays the medical document (Ts) on the display unit (14) by using the other expression vector.

8. A learning device comprising:
a learning unit (26) that learns a learning model to output a medical document (Ts) including features related to a target medical image (G0) by using a plurality of pieces of learning data and generates a trained model (M3) in a case where a feature vector (C0) of the target medical image (G0) and an expression vector (zs) according to feature information including the feature vector (C0) of the target medical image (G0) are input, the plurality of pieces of learning data each including a feature vector (CL0) of a medical image for learning (GL0) and an expression vector (z) of a medical document for learning (TLO), wherein a feature vector represents findings, the findings comprising, for each of a plurality of items, a respective finding, wherein the expression vector (z) represents an expression of the medical document for learning (TL0) and is generated by removing a component related to the feature vector (CL0) from the encoded medical document for learning (TL0) after encoding the medical document for learning (TLO),

9. The learning device according to claim 8, further comprising:
an expression vector generation unit (25) that generates an expression vector (z) of the medical image for learning (GL0) by using the medical document for learning (TL0) and the feature vector (CL0) of the medical image for learning (GL0) corresponding to the medical document for learning (TL0).

10. A medical document creation method performed by a medical document creation apparatus including:
a document creation unit (23) having a trained model (M3) that has been trained to output a medical document including features related to a target medical image (G0) in a case where a feature vector (C0, Ci ) of the target medical image (G0) and an expression vector (z, zs, zi) according to feature information including the feature vector (C0, Ci ) of the target medical image (G0) are input, wherein the trained model (M3) has been learned by using a plurality of pieces of learning data each including a feature vector (CL0) of a medical image for learning (GL0) and an expression vector (z) of a medical document for learning (TLO), wherein a feature vector represents findings, the findings comprising, for each of a plurality of items, a respective finding; and
a storage unit (13) that stores a plurality of expression vectors (z1,z2,z3...) corresponding to feature vectors (CL0) of each of a plurality of the medical images for learning (GL0), wherein each expression vector (z1,z2,z3...) represents the expression of the medical document for learning (TL0) and is generated by removing a component related to the feature vector (CL0) from the encoded medical document for learning (TL0) after encoding the medical document for learning (TLO), the medical document creation method comprising:
selecting at least one expression vector (zs) according to the feature information from the plurality of expression vectors (z1,z2,z3...) stored in the storage unit (13), inputting the feature vector (C0) of the target medical image (G0) and the selected at least one expression vector (zs) into the trained model (M3), and creating at least one medical document (Ts) including features related to the target medical image (G0) in a case where the feature information including the feature vector (C0) of the target medical image (G0) is input.

11. A learning method comprising:
learning a learning model to output a medical document (Ts) including features related to a target medical image (G0) in a case where a feature vector (C0) of the target medical image (G0) and an expression vector (zs) according to feature information including the feature vector (C0) of the target medical image (G0) are input, the learning being by using a plurality of pieces of learning data and generating a trained model (M3), the plurality of pieces of learning data each including a feature vector (CL0) of a medical image for learning (GL0) and an expression vector (z) of a medical document for learning (TLO), wherein a feature vector (CL0,C0) represents findings, the findings comprising, for each of a plurality of items, a respective finding, wherein the expression vector (z) represents an expression of the medical document for learning (TL0) and is generated by removing a component related to the feature vector (CL0) from the encoded medical document for learning (TL0) after encoding the medical document for learning (TLO),

12. A computer-readable storage medium that stores a medical document creation program causing a computer to execute a medical document creation method performed by a medical document creation apparatus including:
a document creation unit (23) having a trained model (M3) that has been trained to output a medical document including features related to a target medical image (G0) in a case where a feature vector (C0) of the target medical image (G0) and an expression vector (zs) according to feature information including the feature vector (C0) of the target medical image (G0) are input, the trained model (M3) having been learned by using a plurality of pieces of learning data each including a feature vector (CL0) of a medical image for learning (GL0) and an expression vector (z) of a medical document for learning (TLO), wherein a feature vector (CL0,C0) represents findings, the findings comprising, for each of a plurality of items, a respective finding; and
a storage unit (13) that stores a plurality of expression vectors ( z1,z2,z3...) corresponding to feature vectors (CL0) of each of a plurality of the medical images for learning (GL0), wherein each expression vector ( z1,z2,z3...) represents the expression of the medical document for learning (TL0) and is generated by removing a component related to the feature vector (CL0) from the encoded medical document for learning (TL0) after encoding the medical document for learning (TLO), the medical document creation program causing the computer to execute a procedure comprising:
selecting at least one expression vector (zs) according to the feature information from the plurality of expression vectors ( z1,z2,z3...) stored in the storage unit (13), inputting the feature vector (C0) of the target medical image (G0) and the selected at least one expression vector (zs) into the trained model (M3), and creating at least one medical document (Ts) including features related to the target medical image (G0) in a case where the feature information including the feature vector (C0) of the target medical image (G0) is input.

13. A computer-readable storage medium that stores a learning program causing a computer to execute a procedure comprising:
learning a learning model to output a medical document (Ts) including features related to a target medical image (G0) in a case where a feature vector (C0) of the target medical image (G0) and an expression vector (zs) according to feature information including the feature vector (C0) of the target medical image (G0) are input, the learning being by using a plurality of pieces of learning data and generating a trained model (M3), the plurality of pieces of learning data each including a feature vector (CL0) of a medical image for learning (GL0) and a medical document for learning (TLO), wherein a feature vector (CL0,C0) represents findings, the findings comprising, for each of a plurality of items, a respective finding, wherein the expression vector (z) represents an expression of the medical document for learning (TL0) and is generated by removing a component related to the feature vector (CL0) from the encoded medical document for learning (TL0) after encoding the medical document for learning (TL0).

## Patentansprüche

1. Vorrichtung (10) zum Erstellen medizinischer Dokumente, umfassend:
eine Dokumenterstellungseinheit (23), die ein trainiertes Modell (M3) aufweist, das trainiert worden ist, um ein medizinisches Dokument (Ts), das Merkmale enthält, die auf ein medizinisches Zielbild (G0) bezogen sind, in einem Fall auszugeben, in dem ein Merkmalsvektor (C0) des medizinischen Zielbildes (G0) und ein Ausdrucksvektor (zs) gemäß Merkmalsinformationen, die den Merkmalsvektor (C0) des medizinischen Zielbildes (G0) enthalten, eingegeben werden, wobei das trainierte Modell (M3) trainiert worden ist, indem mehrere Lerndaten verwendet worden sind, die jeweils einen Merkmalsvektor (CL0) eines medizinischen Bildes (GL0) zum Lernen und einen Ausdrucksvektor (z) eines medizinischen Dokuments (TL0) zum Lernen enthalten, wobei ein Merkmalsvektor (C0, CL0) Befunde darstellt, wobei die Befunde für jedes von mehreren Elementen einen jeweiligen Befund umfassen; und
eine Speichereinheit (13), die mehrere Ausdrucksvektoren (z1, z2, z3...), die Merkmalsvektoren (CL0) von jedem von mehreren der medizinischen Bilder (GL0) zum Lernen entsprechen, speichert, wobei jeder Ausdrucksvektor (z1, z2, z3...) den Ausdruck des medizinischen Dokuments (TL0) zum Lernen darstellt und durch Entfernen einer Komponente, die auf den Merkmalsvektor (CL0) bezogen ist, aus dem kodierten medizinischen Dokument (TL0) zum Lernen nach Kodieren des medizinischen Dokuments (TL0) zum Lernen erzeugt wird,
wobei in einem Fall, in dem die Merkmalsinformationen, die den Merkmalsvektor (C0) des medizinischen Zielbildes (G0) enthalten, eingegeben werden, die Dokumenterstellungseinheit (23) mindestens einen Ausdrucksvektor (zs) gemäß den Merkmalsinformationen aus den mehreren Ausdrucksvektoren (z1, z2, z3...), die in der Speichereinheit (13) gespeichert sind, auswählt, den Merkmalsvektor (C0) des medizinischen Zielbildes (G0) und den ausgewählten mindestens einen Ausdrucksvektor (zs) in das trainierte Modell (M3) eingibt und mindestens ein medizinisches Dokument (Ts), das Merkmale enthält, die auf das medizinische Zielbild (G0) bezogen sind, erstellt.

2. Vorrichtung (10) zum Erstellen medizinischer Dokumente nach Anspruch 1, wobei der Merkmalsvektor (C0, CL0) Befunde in Bezug auf abnormale Verschattungen, die in dem medizinischen Bild (GL0) zum Lernen und dem medizinischen Zielbild (G0) enthalten sind, enthält.

3. Vorrichtung (10) zum Erstellen medizinischer Dokumente nach Anspruch 1 oder 2, ferner umfassend:
eine Analyseeinheit (22), die das medizinische Zielbild (G0) analysiert, um den Merkmalsvektor (C0, CL0) zu erzeugen.

4. Vorrichtung (10) zum Erstellen medizinischer Dokumente nach einem der Ansprüche 1 bis 3, ferner umfassend:
eine Anzeigesteuereinheit (24), die das mindestens eine medizinische Dokument (Ts) auf einer Anzeigeeinheit (14) anzeigt.

5. Vorrichtung (10) zum Erstellen medizinischer Dokumente nach Anspruch 4, wobei
die Dokumenterstellungseinheit (23) mehrere Ausdrucksvektoren (z, zs, zi) gemäß den Merkmalsinformationen des medizinischen Zielbildes (G0) aus den gespeicherten mehreren Ausdrucksvektoren (z1, z2, z3...) auswählt und mehrere der medizinischen Dokumente (Ts) erstellt, und
die Anzeigesteuereinheit (24) die mehreren medizinischen Dokumente (Ts) auf der Anzeigeeinheit (14) anzeigt.

6. Vorrichtung (10) zum Erstellen medizinischer Dokumente nach Anspruch 5, ferner umfassend:
eine Eingabeeinheit (15), die eine Eingabe zum Auswählen eines spezifischen medizinischen Dokuments (Ts) aus den mehreren medizinischen Dokumenten (Ts) empfängt.

7. Vorrichtung (10) zum Erstellen medizinischer Dokumente nach Anspruch 4 oder 5, ferner umfassend:
eine Ausdrucksvektor-Auswahleinheit (30), die eine Auswahl mindestens eines anderen Ausdrucksvektors als des Ausdrucksvektors (z, zs, zi), der bei Erstellen des medizinischen Dokuments (Ts) verwendet wird, empfängt, wobei die Dokumenterstellungseinheit (23) das medizinische Dokument (Ts) durch Verwenden des anderen Ausdrucksvektors als den ausgewählten mindestens einen Ausdrucksvektor (zs) erzeugt, und
die Anzeigesteuereinheit (24) das medizinische Dokument (Ts) auf der Anzeigeeinheit (14) durch Verwenden des anderen Ausdrucksvektors anzeigt.

8. Lernvorrichtung, umfassend:
eine Lerneinheit (26), die ein Lernmodell lernt, um ein medizinisches Dokument (Ts), das Merkmale enthält, die auf ein medizinisches Zielbild (G0) bezogen sind, durch Verwenden mehrerer Lerndaten auszugeben, und ein trainiertes Modell (M3) in einem Fall erzeugt, in dem ein Merkmalsvektor (C0) des medizinischen Zielbildes (G0) und ein Ausdrucksvektor (zs) gemäß Merkmalsinformationen, die den Merkmalsvektor (C0) des medizinischen Zielbildes (G0) enthalten, eingegeben werden, wobei die mehreren Lerndaten jeweils einen Merkmalsvektor (CL0) eines medizinischen Bildes (GL0) zum Lernen und einen Ausdrucksvektor (z) eines medizinischen Dokuments (TL0) zum Lernen enthalten, wobei ein Merkmalsvektor Befunde darstellt, wobei die Befunde für jedes von mehreren Elementen einen jeweiligen Befund umfassen, wobei der Ausdrucksvektor (z) einen Ausdruck des medizinischen Dokuments (TL0) zum Lernen darstellt und durch Entfernen einer Komponente, die auf den Merkmalsvektor (CL0) bezogen ist, aus dem kodierten medizinischen Dokument (TL0) zum Lernen nach Kodieren des medizinischen Dokuments (TL0) zum Lernen erzeugt wird.

9. Lernvorrichtung nach Anspruch 8, ferner umfassend:
eine Ausdrucksvektor-Erzeugungseinheit (25), die einen Ausdrucksvektor (z) des medizinischen Bildes (GL0) zum Lernen durch Verwenden des medizinischen Dokuments (TL0) zum Lernen und des Merkmalsvektors (CL0) des medizinischen Bildes (GL0) zum Lernen, der dem medizinischen Dokument (TL0) zum Lernen entspricht, erzeugt.

10. Verfahren zum Erstellen medizinischer Dokumente, das durch eine Vorrichtung zum Erstellen medizinischer Dokumente durchgeführt wird, die enthält:
eine Dokumenterstellungseinheit (23), die ein trainiertes Modell (M3) aufweist, das trainiert worden ist, um ein medizinisches Dokument, das Merkmale enthält, die auf ein medizinisches Zielbild (G0) bezogen sind, in einem Fall auszugeben, in dem ein Merkmalsvektor (C0, Ci) des medizinischen Zielbildes (G0) und ein Ausdrucksvektor (z, zs, zi) gemäß Merkmalsinformationen, die den Merkmalsvektor (C0, Ci) des medizinischen Zielbildes (G0) enthalten, eingegeben werden, wobei das trainierte Modell (M3) gelernt worden ist, indem mehrere Lerndaten verwendet worden sind, die jeweils einen Merkmalsvektor (CL0) eines medizinischen Bildes (GL0) zum Lernen und einen Ausdrucksvektor (z) eines medizinischen Dokuments (TL0) zum Lernen enthalten, wobei ein Merkmalsvektor Befunde darstellt, wobei die Befunde für jedes von mehreren Elementen einen jeweiligen Befund umfassen; und
eine Speichereinheit (13), die mehrere Ausdrucksvektoren (z1, z2, z3...), die Merkmalsvektoren (CL0) von jedem von mehreren der medizinischen Bilder (GL0) zum Lernen entsprechen, speichert, wobei jeder Ausdrucksvektor (z1, z2, z3...) den Ausdruck des medizinischen Dokuments (TL0) zum Lernen darstellt und durch Entfernen einer Komponente, die auf den Merkmalsvektor (CL0) bezogen ist, aus dem kodierten medizinischen Dokument (TL0) zum Lernen nach Kodieren des medizinischen Dokuments (TL0) zum Lernen erzeugt wird,
wobei das Verfahren zum Erstellen medizinischer Dokumente umfasst:
Auswählen mindestens eines Ausdrucksvektors (zs) gemäß den Merkmalsinformationen aus den mehreren Ausdrucksvektoren (z1, z2, z3...), die in der Speichereinheit (13) gespeichert sind, Eingeben des Merkmalsvektors (C0) des medizinischen Zielbildes (G0) und des mindestens einen ausgewählten Ausdrucksvektors (zs) in das trainierte Modell (M3) und Erstellen mindestens eines medizinischen Dokuments (Ts), das Merkmale enthält, die auf das medizinische Zielbild (G0) bezogen sind, in einem Fall, in dem die Merkmalsinformationen, die den Merkmalsvektor (C0) des medizinischen Zielbildes (G0) enthalten, eingegeben werden.

11. Lernverfahren, umfassend:
Lernen eines Lernmodells, um ein medizinisches Dokument (Ts), das Merkmale enthält, die auf ein medizinisches Zielbild (G0) bezogen sind, in einem Fall auszugeben, in dem ein Merkmalsvektor (C0) des medizinischen Zielbildes (G0) und
ein Ausdrucksvektor (zs) gemäß Merkmalsinformationen, die den Merkmalsvektor (C0) des medizinischen Zielbildes (G0) enthalten, eingegeben werden, wobei das Lernen durch Verwenden mehrerer Lerndaten erfolgt und ein trainiertes Modell (M3) erzeugt, die mehreren Lerndaten jeweils einen Merkmalsvektor (CL0) eines medizinischen Bildes (GL0) zum Lernen und einen Ausdrucksvektor (z) eines medizinischen Dokuments (TL0) zum Lernen enthalten, wobei ein Merkmalsvektor (CL0, C0) Befunde darstellt, wobei die Befunde für jedes von mehreren Elementen einen jeweiligen Befund umfassen, wobei der Ausdrucksvektor (z) einen Ausdruck des medizinischen Dokuments (TL0) zum Lernen darstellt und durch Entfernen einer Komponente, die auf den Merkmalsvektor (CL0) bezogen ist, aus dem kodierten medizinischen Dokument (TL0) zum Lernen nach Kodieren des medizinischen Dokuments (TL0) zum Lernen erzeugt wird.

12. Computerlesbares Speichermedium, das ein Programm zum Erstellen medizinischer Dokumente speichert, das einen Computer veranlasst, ein Verfahren zum Erstellen medizinischer Dokumente auszuführen, das durch eine Vorrichtung zum Erstellen medizinischer Dokumente durchgeführt wird, die enthält:
eine Dokumenterstellungseinheit (23), die ein trainiertes Modell (M3) aufweist, das trainiert worden ist, um ein medizinisches Dokument, das Merkmale enthält, die auf ein medizinisches Zielbild (G0) bezogen sind, in einem Fall auszugeben, in dem ein Merkmalsvektor (C0) des medizinischen Zielbildes (G0) und ein Ausdrucksvektor (zs) gemäß Merkmalsinformationen, die den Merkmalsvektor (C0) des medizinischen Zielbildes (G0) enthalten, eingegeben werden, wobei das trainierte Modell (M3) gelernt worden ist, indem mehrere Lerndaten verwendet worden sind, die jeweils einen Merkmalsvektor (CL0) eines medizinischen Bildes (GL0) zum Lernen und einen Ausdrucksvektor (z) eines medizinischen Dokuments (TL0) zum Lernen enthalten, wobei ein Merkmalsvektor (CL0, C0) Befunde darstellt, wobei die Befunde für jedes von mehreren Elementen einen jeweiligen Befund umfassen; und
eine Speichereinheit (13), die mehrere Ausdrucksvektoren (z1, z2, z3...), die Merkmalsvektoren (CL0) von jedem von mehreren der medizinischen Bilder (GL0) zum Lernen entsprechen, speichert, wobei jeder Ausdrucksvektor (z1, z2, z3...) den Ausdruck des medizinischen Dokuments (TL0) zum Lernen darstellt und durch Entfernen einer Komponente, die auf den Merkmalsvektor (CL0) bezogen ist, aus dem kodierten medizinischen Dokument (TL0) zum Lernen nach Kodieren des medizinischen Dokuments (TL0) zum Lernen erzeugt wird,
wobei das Programm zum Erstellen medizinischer Dokumente den Computer veranlasst, einen Ablauf auszuführen, der umfasst:
Auswählen mindestens eines Ausdrucksvektors (zs) gemäß den Merkmalsinformationen aus den mehreren Ausdrucksvektoren (z1, z2, z3...), die in der Speichereinheit (13) gespeichert sind, Eingeben des Merkmalsvektors (C0) des medizinischen Zielbildes (G0) und des mindestens einen ausgewählten Ausdrucksvektors (zs) in das trainierte Modell (M3) und Erstellen mindestens eines medizinischen Dokuments (Ts), das Merkmale enthält, die auf das medizinische Zielbild (G0) bezogen sind, in einem Fall, in dem die Merkmalsinformationen, die den Merkmalsvektor (C0) des medizinischen Zielbildes (G0) enthalten, eingegeben werden.

13. Computerlesbares Speichermedium, das ein Lernprogramm speichert, das einen Computer veranlasst, einen Ablauf auszuführen, der umfasst:
Lernen eines Lernmodells, um ein medizinisches Dokument (Ts), das Merkmale enthält, die auf ein medizinisches Zielbild (G0) bezogen sind, in einem Fall auszugeben, in dem ein Merkmalsvektor (C0) des medizinischen Zielbildes (G0) und ein Ausdrucksvektor (zs) gemäß Merkmalsinformationen, die den Merkmalsvektor (C0) des medizinischen Zielbildes (G0) enthalten, eingegeben werden, wobei das Lernen durch Verwenden mehrerer Lerndaten erfolgt und ein trainiertes Modell (M3) erzeugt, die mehreren Lerndaten jeweils einen Merkmalsvektor (CL0) eines medizinischen Bildes (GL0) zum Lernen und ein medizinisches Dokument (TL0) zum Lernen enthalten, wobei ein Merkmalsvektor (CL0, C0) Befunde darstellt, wobei die Befunde für jedes von mehreren Elementen einen jeweiligen Befund umfassen, wobei der Ausdrucksvektor (z) einen Ausdruck des medizinischen Dokuments (TL0) zum Lernen darstellt und durch Entfernen einer Komponente, die auf den Merkmalsvektor (CL0) bezogen ist, aus dem kodierten medizinischen Dokument (TL0) zum Lernen nach Kodieren des medizinischen Dokuments (TL0) zum Lernen erzeugt wird.

## Revendications

1. Appareil de création de documents médicaux (10) comprenant :
une unité de création de documents (23) comportant un modèle entraîné (M3) qui a été entraîné pour produire un document médical (Ts) incluant des caractéristiques liées à une image médicale cible (G0) dans un cas où un vecteur de caractéristiques (C0) de l'image médicale cible (G0) et un vecteur d'expression (zs) selon des informations de caractéristiques incluant le vecteur de caractéristiques (C0) de l'image médicale cible (G0) sont entrés, où le modèle entraîné (M3) a été entraîné en utilisant une pluralité d'éléments de données d'apprentissage incluant chacun un vecteur de caractéristiques (CL0) d'une image médicale d'apprentissage (GL0) et un vecteur d'expression (z) d'un document médical d'apprentissage (TL0), où un vecteur de caractéristiques (C0, CL0) représente des constatations, les constatations comprenant, pour chacun d'une pluralité d'éléments, une constatation respective ; et
une unité de stockage (13) qui stocke une pluralité de vecteurs d'expression (z1, z2, z3...) correspondant à des vecteurs de caractéristiques (CL0) de chacune d'une pluralité d'images médicales d'apprentissage (GL0), où chaque vecteur d'expression (z1, z2, z3...) représente l'expression du document médical d'apprentissage (TL0) et est généré en supprimant une composante liée au vecteur de caractéristiques (CL0) du document médical d'apprentissage encodé (TL0) après encodage du document médical d'apprentissage (TL0),
dans lequel, dans un cas où les informations de caractéristiques incluant le vecteur de caractéristiques (C0) de l'image médicale cible (G0) sont entrées, l'unité de création de documents (23) sélectionne au moins un vecteur d'expression (zs) selon les informations de caractéristiques parmi la pluralité de vecteurs d'expression (z1, z2, z3...) stockés dans l'unité de stockage (13), entre le vecteur de caractéristiques (C0) de l'image médicale cible (G0) et l'au moins un vecteur d'expression (zs) sélectionné dans le modèle entraîné (M3), et crée au moins un document médical (Ts) incluant des caractéristiques liées à l'image médicale cible (G0).

2. Appareil de création de documents médicaux (10) selon la revendication 1, dans lequel le vecteur de caractéristiques (C0, CL0) inclut des constatations concernant des ombres anormales incluses dans l'image médicale d'apprentissage (GL0) et l'image médicale cible (G0).

3. Appareil de création de documents médicaux (10) selon la revendication 1 ou la revendication 2, comprenant en outre :
une unité d'analyse (22) qui analyse l'image médicale cible (G0) pour générer le vecteur de caractéristiques (C0, CL0).

4. Appareil de création de documents médicaux (10) selon l'une quelconque des revendications 1 à 3, comprenant en outre :
une unité de contrôle d'affichage (24) qui affiche l'au moins un document médical (Ts) sur une unité d'affichage (14).

5. Appareil de création de documents médicaux (10) selon la revendication 4, dans lequel l'unité de création de documents (23) sélectionne une pluralité de vecteurs d'expression (z, zs, zi) selon les informations de caractéristiques de l'image médicale cible (G0) parmi la pluralité de vecteurs d'expression stockés (z1, z2, z3...) et crée une pluralité des documents médicaux (Ts), et
l'unité de contrôle d'affichage (24) affiche la pluralité de documents médicaux (Ts) sur l'unité d'affichage (14).

6. Appareil de création de documents médicaux (10) selon la revendication 5, comprenant en outre :
une unité d'entrée (15) qui reçoit une entrée pour sélectionner un document médical spécifique (Ts) parmi la pluralité de documents médicaux (Ts).

7. Appareil de création de documents médicaux (10) selon la revendication 4 ou la revendication 5, comprenant en outre :
une unité de sélection de vecteurs d'expression (30) qui reçoit une sélection d'au moins un autre vecteur d'expression autre que le vecteur d'expression (z, zs, zi) utilisé pour créer le document médical (Ts), dans lequel
l'unité de création de documents (23) génère le document médical (Ts) en utilisant l'autre vecteur d'expression comme ledit au moins un vecteur d'expression sélectionné (zs), et
l'unité de contrôle d'affichage (24) affiche le document médical (Ts) sur l'unité d'affichage (14) en utilisant l'autre vecteur d'expression.

8. Dispositif d'apprentissage comprenant :
une unité d'apprentissage (26) qui apprend un modèle d'apprentissage pour produire un document médical (Ts) incluant des caractéristiques liées à une image médicale cible (G0) en utilisant une pluralité d'éléments de données d'apprentissage et qui génère un modèle entraîné (M3) dans un cas où un vecteur de caractéristiques (C0) de l'image médicale cible (G0) et un vecteur d'expression (zs) selon des informations de caractéristiques incluant le vecteur de caractéristiques (C0) de l'image médicale cible (G0) sont entrés, la pluralité d'éléments de données d'apprentissage incluant chacun un vecteur de caractéristiques (CL0) d'une image médicale d'apprentissage (GL0) et un vecteur d'expression (z) d'un document médical d'apprentissage (TL0), où un vecteur de caractéristiques représente des constatations, les constatations comprenant, pour chacun d'une pluralité d'éléments, une constatation respective, où le vecteur d'expression (z) représente une expression du document médical d'apprentissage (TL0) et est généré en supprimant une composante liée au vecteur de caractéristiques (CL0) du document médical d'apprentissage encodé (TL0) après encodage du document médical d'apprentissage (TL0).

9. Dispositif d'apprentissage selon la revendication 8, comprenant en outre :
une unité de génération de vecteurs d'expression (25) qui génère un vecteur d'expression (z) de l'image médicale d'apprentissage (GL0) en utilisant le document médical d'apprentissage (TL0) et le vecteur de caractéristiques (CL0) de l'image médicale d'apprentissage (GL0) correspondant au document médical d'apprentissage (TL0).

10. Procédé de création de documents médicaux effectué par un appareil de création de documents médicaux incluant :
une unité de création de documents (23) comportant un modèle entraîné (M3) qui a été entraîné pour produire un document médical incluant des caractéristiques liées à une image médicale cible (G0) dans un cas où un vecteur de caractéristiques (C0, Ci) de l'image médicale cible (G0) et un vecteur d'expression (z, zs, zi) selon des informations de caractéristiques incluant le vecteur de caractéristiques (C0, Ci) de l'image médicale cible (G0) sont entrés, où le modèle entraîné (M3) a été appris en utilisant une pluralité d'éléments de données d'apprentissage incluant chacun un vecteur de caractéristiques (CL0) d'une image médicale d'apprentissage (GL0) et un vecteur d'expression (z) d'un document médical d'apprentissage (TL0), où un vecteur de caractéristiques représente des constatations, les constatations comprenant, pour chacun d'une pluralité d'éléments, une constatation respective ; et
une unité de stockage (13) qui stocke une pluralité de vecteurs d'expression (z1, z2, z3...) correspondant à des vecteurs de caractéristiques (CL0) de chacune d'une pluralité d'images médicales d'apprentissage (GL0), où chaque vecteur d'expression (z1, z2, z3...) représente l'expression du document médical d'apprentissage (TL0) et est généré en supprimant une composante liée au vecteur de caractéristiques (CL0) du document médical d'apprentissage encodé (TL0) après encodage du document médical d'apprentissage (TL0),
le procédé de création de documents médicaux comprenant :
sélectionner au moins un vecteur d'expression (zs) selon les informations de caractéristiques parmi la pluralité de vecteurs d'expression (z1, z2, z3...) stockés dans l'unité de stockage (13), entrer le vecteur de caractéristiques (C0) de l'image médicale cible (G0) et l'au moins un vecteur d'expression (zs) sélectionné dans le modèle entraîné (M3), et créer au moins un document médical (Ts) incluant des caractéristiques liées à l'image médicale cible (G0) dans un cas où les informations de caractéristiques incluant le vecteur de caractéristiques (C0) de l'image médicale cible (G0) sont entrées.

11. Procédé d'apprentissage comprenant :
apprendre un modèle d'apprentissage pour produire un document médical (Ts) incluant des caractéristiques liées à une image médicale cible (G0) dans un cas où un vecteur de caractéristiques (C0) de l'image médicale cible (G0) et un vecteur d'expression (zs) selon des informations de caractéristiques incluant le vecteur de caractéristiques (C0) de l'image médicale cible (G0) sont entrés, l'apprentissage étant effectué en utilisant une pluralité d'éléments de données d'apprentissage et en générant un modèle entraîné (M3), la pluralité d'éléments de données d'apprentissage incluant chacun un vecteur de caractéristiques (CL0) d'une image médicale d'apprentissage (GL0) et un vecteur d'expression (z) d'un document médical d'apprentissage (TL0), où un vecteur de caractéristiques (CL0, C0) représente des constatations, les constatations comprenant, pour chacun d'une pluralité d'éléments, une constatation respective, où le vecteur d'expression (z) représente une expression du document médical d'apprentissage (TL0) et est généré en supprimant une composante liée au vecteur de caractéristiques (CL0) du document médical d'apprentissage encodé (TL0) après encodage du document médical d'apprentissage (TL0).

12. Support de stockage lisible par ordinateur qui stocke un programme de création de documents médicaux amenant un ordinateur à exécuter un procédé de création de documents médicaux effectué par un appareil de création de documents médicaux incluant :
une unité de création de documents (23) comportant un modèle entraîné (M3) qui a été entraîné pour produire un document médical incluant des caractéristiques liées à une image médicale cible (G0) dans un cas où un vecteur de caractéristiques (C0) de l'image médicale cible (G0) et un vecteur d'expression (zs) selon des informations de caractéristiques incluant le vecteur de caractéristiques (C0) de l'image médicale cible (G0) sont entrés, le modèle entraîné (M3) ayant été appris en utilisant une pluralité d'éléments de données d'apprentissage incluant chacun un vecteur de caractéristiques (CL0) d'une image médicale d'apprentissage (GL0) et un vecteur d'expression (z) d'un document médical d'apprentissage (TL0), où un vecteur de caractéristiques (CL0, C0) représente des constatations, les constatations comprenant, pour chacun d'une pluralité d'éléments, une constatation respective ; et
une unité de stockage (13) qui stocke une pluralité de vecteurs d'expression (z1, z2, z3...) correspondant à des vecteurs de caractéristiques (CL0) de chacune d'une pluralité d'images médicales d'apprentissage (GL0), où chaque vecteur d'expression (z1, z2, z3...) représente l'expression du document médical d'apprentissage (TL0) et est généré en supprimant une composante liée au vecteur de caractéristiques (CL0) du document médical d'apprentissage encodé (TL0) après encodage du document médical d'apprentissage (TL0),
le programme de création de documents médicaux amenant l'ordinateur à exécuter une procédure comprenant :
sélectionner au moins un vecteur d'expression (zs) selon les informations de caractéristiques parmi la pluralité de vecteurs d'expression (z1, z2, z3...) stockés dans l'unité de stockage (13), entrer le vecteur de caractéristiques (C0) de l'image médicale cible (G0) et l'au moins un vecteur d'expression (zs) sélectionné dans le modèle entraîné (M3), et créer au moins un document médical (Ts) incluant des caractéristiques liées à l'image médicale cible (G0) dans un cas où les informations de caractéristiques incluant le vecteur de caractéristiques (C0) de l'image médicale cible (G0) sont entrées.

13. Support de stockage lisible par ordinateur qui stocke un programme d'apprentissage amenant un ordinateur à exécuter une procédure comprenant :
apprendre un modèle d'apprentissage pour produire un document médical (Ts) incluant des caractéristiques liées à une image médicale cible (G0) dans un cas où un vecteur de caractéristiques (C0) de l'image médicale cible (G0) et un vecteur d'expression (zs) selon des informations de caractéristiques incluant le vecteur de caractéristiques (C0) de l'image médicale cible (G0) sont entrés, l'apprentissage étant effectué en utilisant une pluralité d'éléments de données d'apprentissage et en générant un modèle entraîné (M3), la pluralité d'éléments de données d'apprentissage incluant chacun un vecteur de caractéristiques (CL0) d'une image médicale d'apprentissage (GL0) et un document médical d'apprentissage (TL0), où un vecteur de caractéristiques (CL0, C0) représente des constatations, les constatations comprenant, pour chacun d'une pluralité d'éléments, une constatation respective, où le vecteur d'expression (z) représente une expression du document médical d'apprentissage (TL0) et est généré en supprimant une composante liée au vecteur de caractéristiques (CL0) du document médical d'apprentissage encodé (TL0) après encodage du document médical d'apprentissage (TL0).
